# EUROPEAN PATENT SPECIFICATION

(11) **EP 2 918 999 B1**
(45) Date of publication and mention of the grant of the patent: **06.06.2018**
(21) Application number: 13853891.3
(22) Date of filing: 05.11.2013
(51) Int. Cl.: G01N 33/487

(54) **INSPECTION DEVICE FOR BIOLOGICALLY DERIVED MATERIAL**
VORRICHTUNG ZUM PRÜFEN EINES BIOLOGISCH ABGELEITETEN MATERIALS
DISPOSITIF D'INSPECTION DE MATÉRIAU D'ORIGINE BIOLOGIQUE

(30) Priority: 06.11.2012 JP 2012244171
(43) Date of publication of application: 16.09.2015
(73) Proprietor: Panasonic Intellectual Property Management Co., Ltd., Osaka-shi, Osaka 540-6207 (JP)
(72) Inventor: NAKATANI, Masaya, Osaka-shi, Osaka 540-6207 (JP); OKA, Hiroaki, Osaka-shi, Osaka 540-6207 (JP); YAMAMOTO, Takeki, Osaka-shi, Osaka 540-6207 (JP)
(74) Representative: Vigand, Philippe
(86) International application number: PCT/JP2013/006505
(87) International publication number: WO 2014/073195

(56) References cited:
- WO-A1-02/103354
- WO-A1-2009/034697
- WO-A1-2011/142117
- JP-A- 2002 523 726
- JP-A- 2004 166 692
- JP-A- 2005 156 234
- JP-A- 2005 265 758
- JP-A- 2005 537 498
- JP-A- 2007 198 876
- JP-A- 2007 298 351
- JP-A- 2008 039 624
- US-A1- 2011 045 582
- US-A1- 2012 261 257
- US-A1- 2012 267 260

## Description

### TECHNICAL FIELD

The technical field relates to an examination device for a substance of biological origin, which is for use in examination and analysis of activity states of substances of biological origin such as cells, tissue, and embryos.

### BACKGROUND ART

Substances of biological origin such as cells, tissue, and embryos are active in conveying various substances. For example, cardiac muscle cells convey, for instance, K ions, Na ions, and Ca ions, thus transmitting information using electrical signals and compounds and controlling pulsation of the heart. Segmentation of an embryo consumes oxygen in the vicinity.

In order to observe activity states of substances of biological origin, there is a method for detecting a physicochemical change which occurs around a substance of biological origin while the substance of biological origin is held in an examination device. This method is used to conduct, using a model cell, a pharmacological test on a compound that is a candidate for a new drug, and to examine the activity of an embryo.

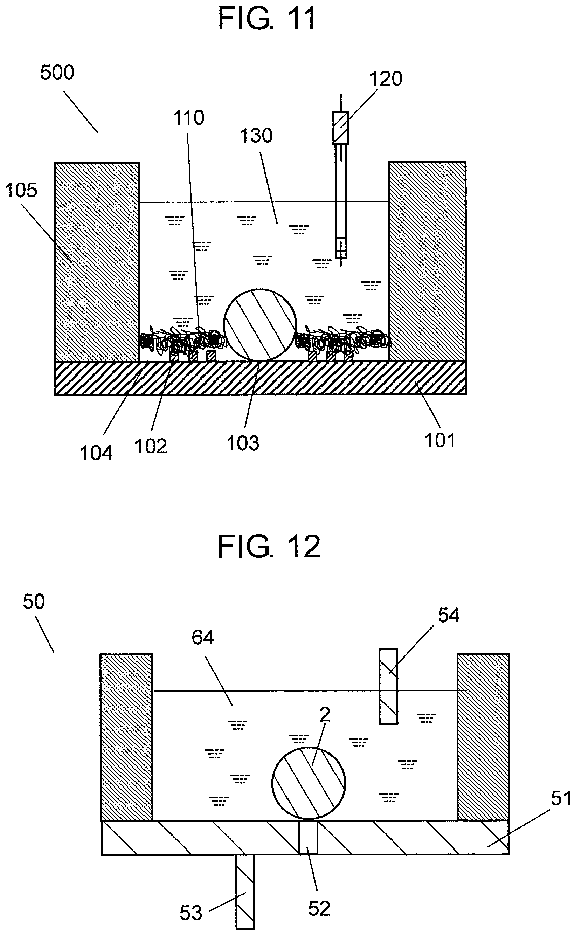

FIG. 12 is a vertical cross-sectional view of conventional examination device 50 for substance 2 of biological origin. Here, substance 2 of biological origin is a cell, for example. Examination device 50 is an electrophysiological sensor device which examines an activity state of a cell.

Substance 2 of biological origin is held at through hole 52. Through hole 52 is smaller than substance 2 of biological origin which is to be observed, and has a diameter of two micrometers, for example.

Monitor electrode 53 and reference electrode 54 are disposed in two areas divided by diaphragm 51. Examination device 50 is filled with culture solution 64.

The electrical property of a cell can be studied and the activity state of the cell can be examined and analyzed by measuring, for instance, a current flowing between and a potential difference between monitor electrode 53 and reference electrode 54.

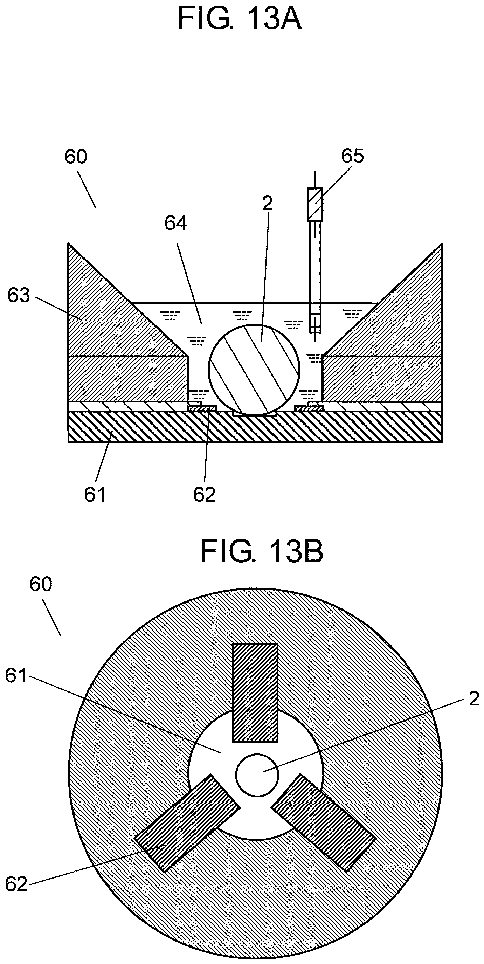

FIG. 13A is a vertical cross-sectional view of conventional examination device 60 for substance 2 of biological origin. FIG. 13B is a horizontal cross-sectional view of conventional examination device 60 for substance 2 of biological origin. Here, substance 2 of biological origin is an embryo, for example. Examination device 60 is an embryo monitoring device which measures an amount of oxygen dissolved around an embryo, to examine the activity state of the embryo. Examination device 60 has base 61 for placing substance 2 of biological origin, at a bottom portion of container 63. In addition, monitor electrodes 62 are disposed on base 61. Container 63 is filled with appropriate culture solution 64 for maintaining an embryo healthy. Reference electrode 65 is disposed in culture solution 64. Potential differences and currents between reference electrode 65 and monitor electrodes 62 are measured to obtain an amount of dissolved oxygen. The amount of dissolved oxygen relates to the amount of oxygen consumed as a result of the activity conducted by an embryo, and thus the activity state of the embryo can be observed by obtaining the amount of dissolved oxygen.

It should be noted that prior art documents with regard to this application include a Patent Literature and a Non-Patent Literature below.

### Citation List

### Patent Literature

Patent Literature 1: Japanese Unexamined Patent Application Publication No. 2005-156234
Non-Patent Literature 1: "Monitoring oxygen consumption of single mouse embryos using an integrated electrochemical microdevice" Biosensors and Bioelectronics 30 (2011) 100-106

Further art is disclosed by the document US 2012/0267260.

### SUMMARY OF THE INVENTION

The present invention concerns a combination of at least one instrumentation amplifier and an examination device for a substance of biological origin, as defined in the appended claims. An examination device for a substance of biological origin includes: a base; a placing area for placing the substance of biological origin, the placing area being on a first surface of the base; a first monitor electrode; and a second monitor electrode. A shortest distance from a center of the placing area to the first monitor electrode and a shortest distance from the center of the placing area to the second monitor electrode are different.

### BRIEF DESCRIPTION OF DRAWINGS

FIG. 1A is a vertical cross-sectional view of an examination device for a substance of biological origin according to a first exemplary embodiment.
FIG. 1B is a horizontal cross-sectional view of the examination device for the substance of biological origin according to the first exemplary embodiment.
FIG. 2A is a vertical cross-sectional view of an examination device for a substance of biological origin according to a second exemplary embodiment.
FIG. 2B is a vertical cross-sectional view of another examination device for a substance of biological origin according to the second exemplary embodiment.
FIG. 3 is a vertical cross-sectional view of another examination device for a substance of biological origin not within the scope of the present invention.
FIG. 4A is a vertical cross-sectional view of an examination device for a substance of biological origin according to a third exemplary embodiment.
FIG. 4B is a horizontal cross-sectional view of the examination device for the substance of biological origin according to the third exemplary embodiment.
FIG. 5 is a vertical cross-sectional view of another examination device for a substance of biological origin according to the third exemplary embodiment.
FIG. 6 is a horizontal cross-sectional view of another examination device for a substance of biological origin according to the third exemplary embodiment.
FIG. 7 is a vertical cross-sectional view of another examination device for a substance of biological origin not within the scope of the present invention.
FIG. 8 is a vertical cross-sectional view of another examination device for a substance of biological origin not within the scope of the present invention.
FIG. 9 is a vertical cross-sectional view of an examination device for a substance of biological origin according to a fourth exemplary embodiment.
FIG. 10 is a vertical cross-sectional view of another examination device for a substance of biological origin according to the fourth exemplary embodiment.
FIG. 11 is a vertical cross-sectional view of an examination device for a substance of biological origin according to a fifth exemplary embodiment.
FIG. 12 is a vertical cross-sectional view of a conventional examination device for a substance of biological origin.
FIG. 13A is a vertical cross-sectional view of another conventional examination device for a substance of biological origin.
FIG. 13B is a horizontal cross-sectional view of the other conventional examination device for the substance of biological origin.

### DESCRIPTION OF EMBODIMENTS

Conventional examination devices 50 and 60 are not suitable for measuring physicochemical changes occurring around substance 2 of biological origin in a spatially resolved manner. Specifically, monitor electrode 53 and reference electrode 54 of conventional examination device 50 are in a one-to-one relation. Thus, the distance from monitor electrode 53 to substance 2 of biological origin is fixed. Further, in examination device 60, the distances from monitor electrodes 62 to substance 2 of biological origin are the same.

Accordingly, in order to measure changes in a spatially resolved manner, monitor electrodes 53 and 62 need to be brought close to or away from substance 2 of biological origin which is to be observed. However, this may cause damage on substance 2 of biological origin with a monitor electrode, and result in variations in measurement. Thus, monitor electrodes 53 and 62 need to be operated carefully and highly accurately. Accordingly, a highly precise device for controlling the position of an electrode is necessary, and considerable skills are required when such a device is handled.

### FIRST EXEMPLARY EMBODIMENT

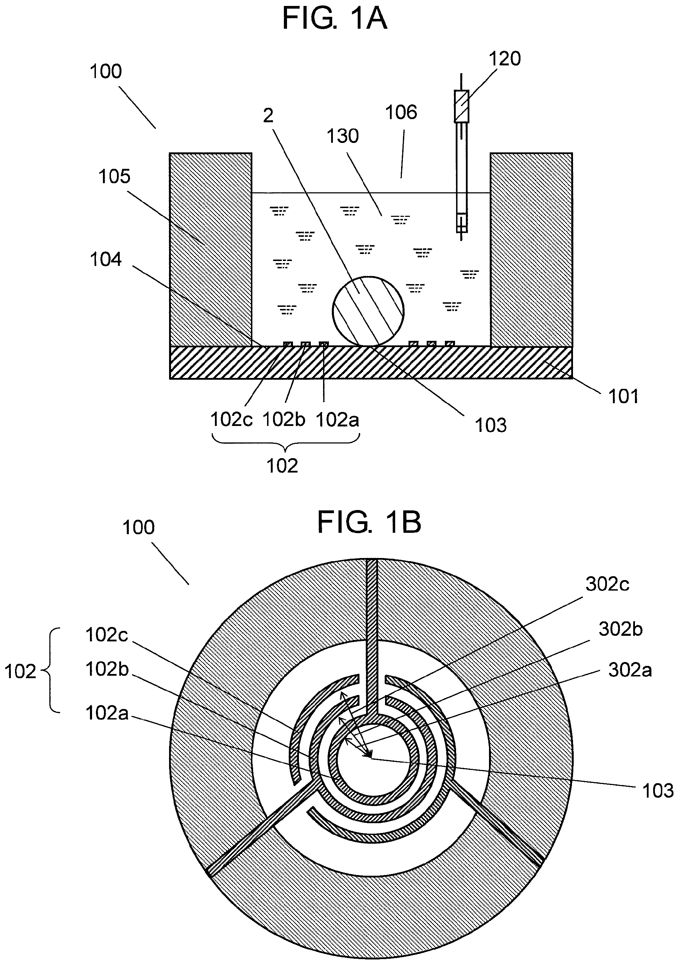

FIG. 1A is a vertical cross-sectional view of examination device 100 for substance 2 of biological origin according to a first exemplary embodiment. FIG. 1B is a horizontal cross-sectional view of examination device 100 for substance 2 of biological origin according to the first exemplary embodiment.

Examination device 100 for substance 2 of biological origin includes: base 101; placing area 103 for placing substance 2 of biological origin, placing area 103 being on placing surface 104 of base 101; first monitor electrode 102a; and second monitor electrode 102b. The shortest distance from the center of placing area 103 to first monitor electrode 102a differs from the shortest distance from the center of placing area 103 to second monitor electrode 102b. Here, placing surface 104 is a first surface of base 101 on which substance 2 of biological origin is placed. It should be noted that examination device 100 may have third monitor electrode 102c. In other words, two or more types of monitor electrodes 102 may be included.

Examples of substance 2 of biological origin include a cell, tissue, an embryo, and others.

Base 101 is formed, for example, with glass, resin, silicon, ceramics, or the like.

Here, a portion of placing surface 104 of base 101 may be processed into a recess as placing area 103, for example. Alternatively, placing surface 104 may be processed into a shape entirely dented toward the center, to form placing area 103. Even if the cross section of placing surface 104 has a cone angle of about several degrees, the placing area of examination device 100 can be determined, and thus it is preferable to form a dented or recessed portion. It should be noted that for example, such a dented or recessed portion is formed by dry etching.

Monitor electrodes 102a, 102b, and 102c are circularly formed on base 101 in top view (or in other words, when viewed in a direction facing the first surface). Monitor electrodes 102a, 102b, and 102c are formed on the same plane as placing surface 104 for placing substance 2 of biological origin. Monitor electrodes 102 include a plurality of monitor electrodes, namely, monitor electrodes 102a, 102b, and 102c. It is preferable to form monitor electrodes 102 using a precious metal such as platinum, gold, or silver, for example. Furthermore, monitor electrodes 102 may include material typically used as the material of an electrode of a battery, such as carbon, lithium cobalt oxide, or the like. The material of monitor electrodes 102 may be selected, taking into consideration composition of culture solution 130, necessary voltage and current, and others at the time of measurement.

Monitor electrodes 102a, 102b, and 102c are disposed so as to be separate from placing area 103 for placing substance 2 of biological origin by different distances. Here, a distance is the shortest distance from the center of placing area 103 to each of monitor electrode 102a, 102b, and 102c. Thus, shortest distance 302a from the center of placing area 103 to monitor electrode 102a, shortest distance 302b from the center of placing area 103 to monitor electrode 102b, and shortest distance 302c from the center of placing area 103 to monitor electrode 102c are different. In the present embodiment, monitor electrodes 102a, 102b, and 102c are formed in ascending order of the distance from the center of placing area 103.

Monitor electrodes 102a, 102b, and 102c are individually connected to instrumentation amplifiers (not illustrated), and potential differences and currents between reference electrode 120 and monitor electrodes 102a, 102b, and 102c, for instance, are measured individually.

Further, the perimeter of the upper surface of base 101 may be surrounded by wall portion 105. Forming wall portion 105 allows well 106 to be formed inside wall portion 105. Wall portion 105 is formed with, for example, glass, resin, silicon, ceramics, or the like, as with base 101. Forming wall portion 105 achieves a guide for when substance 2 of biological origin is securely placed on placing surface 104, thus facilitating operation. It should be noted that the inner surface of wall portion 105 is preferably subjected to hydrophilic treatment. If the inner surface of wall portion 105 has undergone hydrophilic treatment, solution can be poured into well 106 with ease.

Furthermore, wall portion 105 preferably has a tapered shape so that the opening has a larger diameter than the bottom portion. The tapered shape allows substance 2 of biological origin to be placed more reliably.

The following describes operation of examination device 100.

Culture solution 130 which includes substance 2 of biological origin is poured into well 106, and an embryo which is substance 2 of biological origin is placed on placing area 103.

Reference electrode 120 is put into culture solution 130. It should be noted that Ag/AgCl, Pt, or Au, for instance is used for reference electrode 120.

Here, reference electrode 120 is fixed by a mechanism (not illustrated) provided on the lateral surface of wall portion 105. Fixing reference electrode 120 allows the positional relationship between reference electrode 120 and monitor electrodes 102 to be fixed, making no change in positional relationship each time measurement is performed. Thus, measurement can be performed with sufficient repeatability.

Monitor electrodes 102a, 102b, and 102c are formed on base 101. Currents flowing between reference electrode 120 and monitor electrodes 102a, 102b, and 102c are measured to obtain the amount of dissolved oxygen in culture solution 130. The amount of dissolved oxygen relates to the amount of oxygen consumed as a result of activity conducted by an embryo. Accordingly, the activity state of the embryo can be observed by obtaining an amount of dissolved oxygen.

Monitor electrodes 102a, 102b, and 102c are separate from an embryo (substance 2 of biological origin) by different distances. Thus, it is not necessary to move monitor electrodes 102 to perform measurement as with a conventional monitor electrode, and physicochemical changes can be measured at the positions of monitor electrodes 102a, 102b, and 102c. Further, active oxygen, metabolites, and others from substance 2 of biological origin have radial concentration gradients. Accordingly, the concentration gradient of dissolved oxygen can be measured from currents flowing between reference electrode 120 and monitor electrodes 102a, 102b, and 102c which are separate from substance 2 of biological origin by different distances. As a result, physicochemical changes around substance 2 of biological origin can be measured with ease in a spatially resolved manner.

Monitor electrodes 102a, 102b, and 102c are connected to instrumentation amplifiers, and thus currents at the monitor electrodes can be measured simultaneously. In this manner, amounts of dissolved oxygen indicative of physicochemical changes occurring around substance 2 of biological origin can be simultaneously measured. Furthermore, monitor electrodes 102 may be connected to a single instrumentation amplifier using switches or relays, and the instrumentation amplifier may be time-shared (time-divided). Connecting monitor electrodes 102 to a single instrumentation amplifier using a switching circuit achieves a reduction in the size of the device. In this case, however, switches or relays are necessary which operate at sufficiently high speed with respect to temporal changes in amounts of dissolved oxygen.

It should be noted that if base 101 is a conductor or a semiconductor, it is preferable to dispose an insulating layer (not illustrated) between base 101 and monitor electrodes 102. Furthermore, extracted portions of monitor electrodes 102 which are in contact with an electrolysis solution are preferably covered with an insulating layer. Further, an insulating layer having small holes may be formed on monitor electrodes 102, and monitor electrodes 102 may be exposed from the small holes. In this manner, currents due to electrochemical reaction can be less detected at unnecessary positions. Accordingly, physicochemical changes caused by substance 2 of biological origin can be measured more accurately.

### SECOND EXEMPLARY EMBODIMENT

The following describes examination device 200 according to a second exemplary embodiment with reference to the drawings. In the second exemplary embodiment, the same numerals are assigned to an equivalent configuration to that in the first exemplary embodiment, and a detailed description thereof is omitted.

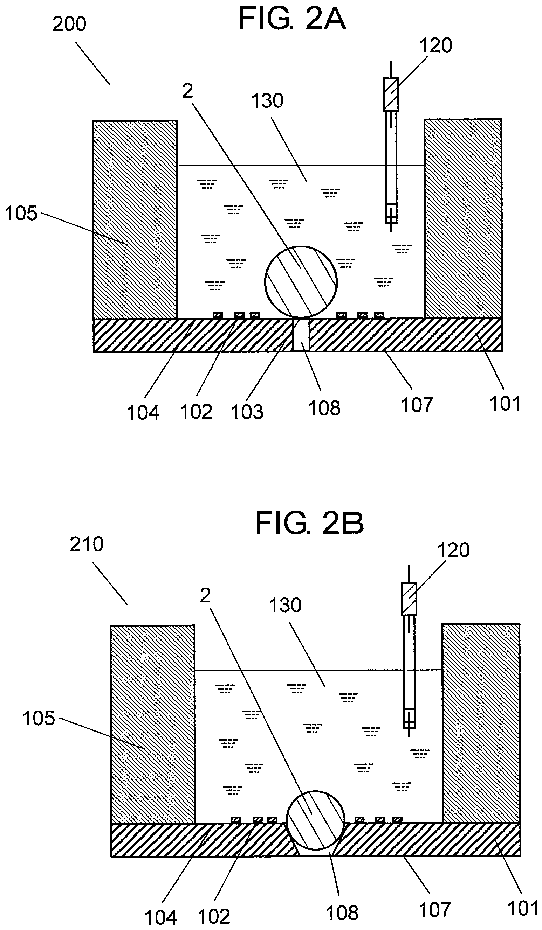

FIG. 2A is a vertical cross-sectional view of examination device 200 according to the second exemplary embodiment. The second exemplary embodiment differs from the first exemplary embodiment in that first through hole 108 is formed in placing area 103 of base 101 on which substance 2 of biological origin is placed. First through hole 108 penetrates through base 101 from placing surface 104 for placing substance 2 of biological origin to opposite surface 107 (second surface) on the opposite side.

First through hole 108 is formed by etching, laser processing, or the like so as to have a diameter smaller than the diameter of substance 2 of biological origin.

Since first through hole 108 is provided as placing area 103 for placing substance 2 of biological origin, substance 2 of biological origin can be accurately placed on first through hole 108 by generating negative pressure on the opposite surface 107 side or positive pressure on the placing surface 104 side, for example. Accordingly, substance 2 of biological origin can be securely placed with ease.

In a state where substance 2 of biological origin is securely placed as descried above, physicochemical changes around substance 2 of biological origin can be measured using monitor electrodes 102 in a spatially resolved manner. Accordingly, a concentration gradient around substance 2 of biological origin can be obtained with ease.

It should be noted that as illustrated in FIG. 2B, first through hole 108 of examination device 210 preferably has a size (diameter) greater on the placing surface 104 side than on the opposite surface 107 side. This can prevent substance 2 of biological origin from being damaged when substance 2 of biological origin comes into contact with the opening of first through hole 108.

It should be noted that in order to prevent dryness in the vicinity of first through hole 108, at least a portion of opposite surface 107 around first through hole 108 is preferably filled with culture solution 130.

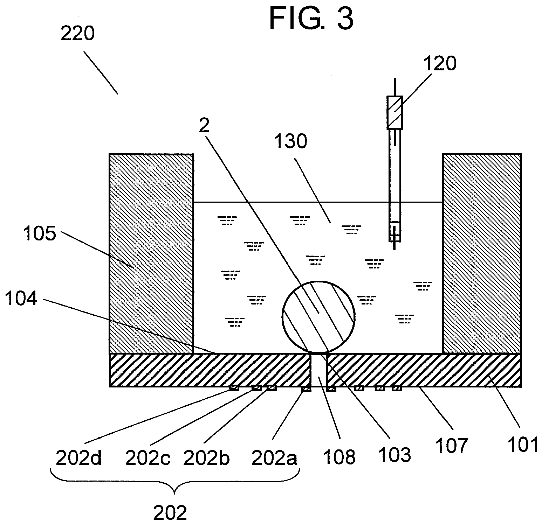

FIG. 3 is a vertical cross-sectional view of examination device 220 not within the scope of the present invention. In FIG. 2A, monitor electrodes 102 are formed on the same plane as placing surface 104 for placing substance 2 of biological origin, whereas monitor electrodes 202 are formed on opposite surface 107 in FIG. 3. Monitor electrodes 202 include monitor electrodes 202a, 202b, 202c, and 202d.

Monitor electrodes 202a, 202b, 202c, and 202d are disposed near and around first through hole 108. Monitor electrodes 202a, 202b, 202c, and 202d are formed so as to be separate from the center of placing area 103 by different distances. Thus, the shortest distances from the center of placing area 103 to monitor electrodes 202a, 202b, 202c, and 202d are different.

Changes caused by the activity conducted by substance 2 of biological origin on the placing surface 104 side (for example, changes in oxygen concentration in culture solution 130) also occur on opposite surface 107 via first through hole 108. Due to the differences in oxygen concentration, current values and potential differences between reference electrode 120 and monitor electrodes 202a, 202b, 202c, and 202d are different. Accordingly, the oxygen concentrations at the positions where monitor electrodes 202a, 202b, 202c, and 202d are disposed can be measured.

Substance 2 of biological origin produces metabolites such as protein, and wastes. Thus, if monitor electrodes 202 are formed on the same plane as placing surface 104 for placing substance 2 of biological origin, the metabolites and wastes from substance 2 of biological origin may adhere to monitor electrodes 202, and make monitor electrodes 202 dirty.

If monitor electrodes 202 are dirty, currents on the surfaces of monitor electrodes 202 may be blocked. Accordingly, the amount of dissolved oxygen may not be accurately measured, and the amount of oxygen consumed by substance 2 of biological origin may not be accurately measured.

As illustrated in FIG. 3, metabolites, for instance, from substance 2 of biological origin can be inhibited from adhering to monitor electrodes 202, by forming monitor electrodes 202 on opposite surface 107 which is on the opposite side of base 101 from placing surface 104 for placing substance 2 of biological origin.

Monitor electrodes 202a, 202b, 202c, and 202d are individually connected to instrumentation amplifiers, and potential differences and currents between reference electrode 120 and monitor electrodes 202a, 202b, 202c, and 202d can be individually measured.

It should be noted that if monitor electrodes 202 are formed on opposite surface 107 of base 101 from placing surface 104, an opening of first through hole 108 on the opposite surface 107 side is preferably larger than a central portion of first through hole 108. As described in the first exemplary embodiment, the size of first through hole 108 is preferably larger on the placing surface 104 side than on the opposite surface 107 side. Furthermore, by making the opening in opposite surface 107 larger than the central portion, physicochemical changes which occur due to the activity conducted by substance 2 of biological origin are quickly diffused and transmitted also to the opposite surface 107 side. Accordingly, measurement can be performed with ease even if monitor electrodes 202 are formed on the opposite side of base 101 from placing surface 104 for placing substance 2 of biological origin.

It should be noted that wall portion 105 is formed so as to be in contact with placing surface 104. However, a wall portion may be formed on opposite surface 107 on the opposite side of base 101 from placing surface 104. In other words, wall portion 105 may be formed on each of placing surface 104 and opposite surface 107.

If monitor electrodes 202 are formed on opposite surface 107, first through hole 108 is preferably short. Thus, base 101 is preferably thin in order to have short first through hole 108. Even if base 101 is thin, the strength of base 101 can be secured by forming a wall portion on opposite surface 107.

### THIRD EXEMPLARY EMBODIMENT

The following describes examination device 300 for a substance of biological origin according to a third exemplary embodiment, with reference to the drawings. In the present embodiment, the same numerals are assigned to an equivalent configuration to that in the first exemplary embodiment, and a detailed description thereof is omitted.

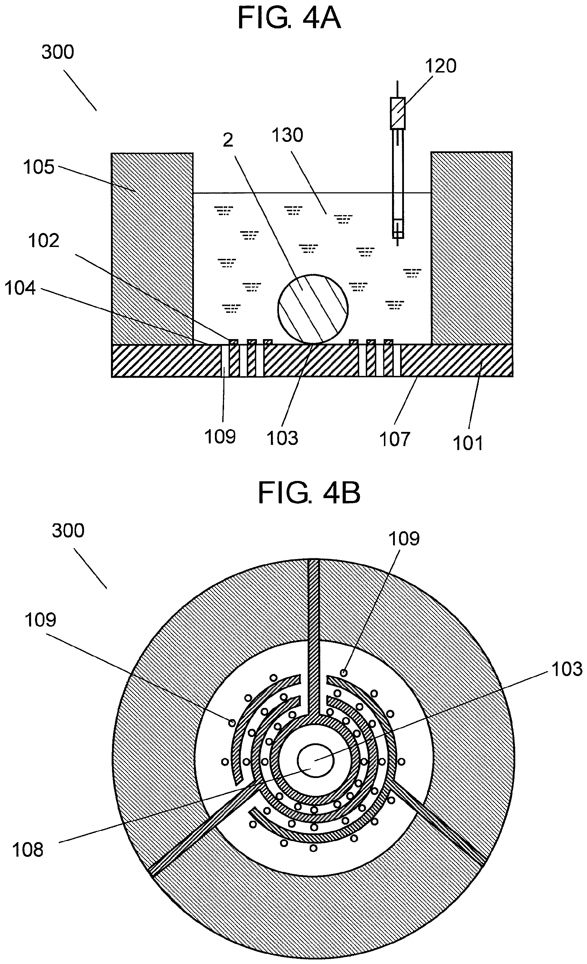

FIG. 4A is a vertical cross-sectional view of examination device 300 for substance 2 of biological origin according to the third exemplary embodiment. FIG. 4B is a horizontal cross-sectional view of examination device 300 for substance 2 of biological origin according to the third exemplary embodiment. The present exemplary embodiment differs from the first exemplary embodiment in that one or more second through holes 109 are formed in an area different from placing area 103 for placing substance 2 of biological origin. One or more second through holes 109 penetrate through base 101 from placing surface 104 to opposite surface 107 on the opposite side of base 101 from placing surface 104. Second through holes 109 are radially formed from placing area 103 for placing substance 2 of biological origin.

Monitor electrodes 102 are formed on the same plane as placing surface 104 for placing substance 2 of biological origin. The shortest distances from the center of placing area 103 to monitor electrodes 102 are different.

Physicochemical changes are caused by the activity conducted by substance 2 of biological origin, and a radial and continuous concentration gradient is formed about substance 2 of biological origin. Supposing that substance 2 of biological origin is placed in the air, an ideal concentration gradient is considered to be formed about substance 2 of biological origin. However, a concentration gradient caused by substance 2 of biological origin may be interrupted by placing surface 104 which is a border plane, if substance 2 of biological origin is placed on placing surface 104.

However, in the present embodiment, second through holes 109 are radially formed in succession, and thus a concentration gradient due to physicochemical changes is continuously formed on the opposite surface 107 side via second through holes 109. As a result, a concentration gradient near placing surface 104 can be measured more accurately.

Second through holes 109 preferably have a diameter smaller than substance 2 of biological origin.

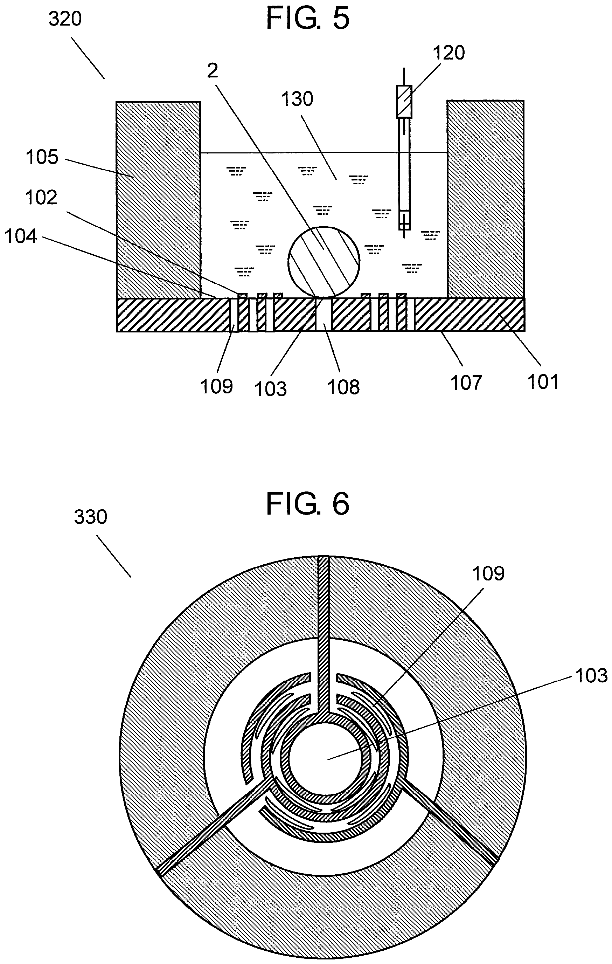

FIG. 5 is a vertical cross-sectional view of examination device 320 for substance 2 of biological origin according to the present embodiment.

Examination device 320 in FIG. 5 differs from examination device 300 in FIGS. 4A and 4B in that through hole 108 is formed in placing area 103 on base 101 at which substance 2 of biological origin is placed. The diameter of second through holes 109 is preferably smaller than the diameter of first through hole 108. By making second through holes 109 smaller than first through hole 108, the velocity of flow of culture solution 130 generated when negative pressure is applied onto the opposite surface 107 side is higher in first through hole 108 than in second through holes 109. Accordingly, the velocity of flow of culture solution 130 generated in second through holes 109 does not prevent secure placement of substance 2 of biological origin. It should be noted that the diameter of second through holes 109 may be larger than the diameter of first through hole 108. However, in that case, if negative pressure is applied onto the opposite surface 107 side, the flow velocity of culture solution 130 is higher in second through holes 109 than in first through hole 108, which requires caution.

FIG. 6 is a horizontal cross-sectional view of examination device 330 for substance 2 of biological origin according to the third exemplary embodiment. As illustrated in FIG. 6, the shape of second through holes 109 may be an ellipse in top view (in other words, in a direction facing a first surface), rather than a circle. In addition, the shape of second through holes 109 may be a crescent along monitor electrodes 202.

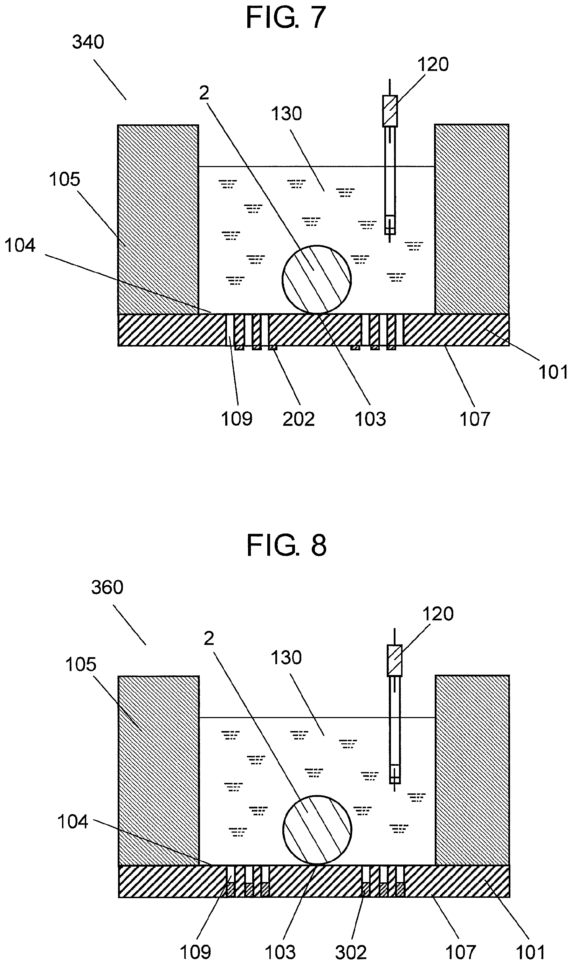

FIG. 7 is a vertical cross-sectional view of examination device 340 for substance 2 of biological origin not within the scope of the present invention. It differs from the first exemplary embodiment in that one or more second through holes 109 are formed in an area different from placing area 103 for placing substance 2 of biological origin, and further in that monitor electrodes 202 are formed on opposite surface 107 on the opposite side of base 101 from placing surface 104 for placing substance 2 of biological origin. The shortest distances from the center of placing area 103 to monitor electrodes 202 are different. In addition, second through holes 109 penetrate through base 101 from placing surface 104 to opposite surface 107 on the opposite side. Furthermore, second through holes 109 are formed radially from placing area 103. This configuration reduces dirt on monitor electrodes 202 due to metabolites from substance 2 of biological origin. In addition, oxygen concentration according to a concentration gradient from second through holes 109 can be measured. Accordingly, active oxygen concentration can be measured still more efficiently.

It should be noted that second through holes 109 and a pattern formed by monitor electrodes 202 may overlap. A concentration gradient based on physicochemical changes caused by substance 2 of biological origin can be measured more accurately at a position closer to placing surface 104. Thus, monitor electrodes 202 are preferably located closer to second through holes 109.

FIG. 8 is a vertical cross-sectional view of examination device 360 for substance 2 of biological origin not within the scope of the present invention.

Monitor electrodes 302 are formed in second through holes 109. Accordingly, monitor electrodes 302 are embedded in part of second through holes 109. Alternatively, monitor electrodes 302 are formed so as to close or fill second through holes 109. The shortest distances from the center of placing area 103 to monitor electrodes 302 are different.

The above configuration allows physicochemical changes caused by substance 2 of biological origin to be measured using monitor electrodes 302 formed at positions close to placing surface 104. On the other hand, the electrode area exposed from placing surface 104 is smaller than that in the first exemplary embodiment. This inhibits influence of dirt generated by the activity of substance 2 of biological origin.

Here, monitor electrodes 302 are formed with a conductive material. It should be noted that in FIGS. 7 and 8, first through hole 108 may be formed in placing area 103 of base 101 at which substance 2 of biological origin is placed.

### FOURTH EXEMPLARY EMBODIMENT

The following describes examination device 400 for substance 2 of biological origin according to a fourth exemplary embodiment, with reference to the drawings. In the present exemplary embodiment, the same numerals are assigned to an equivalent configuration to that in the first exemplary embodiment, and a detailed description thereof is omitted.

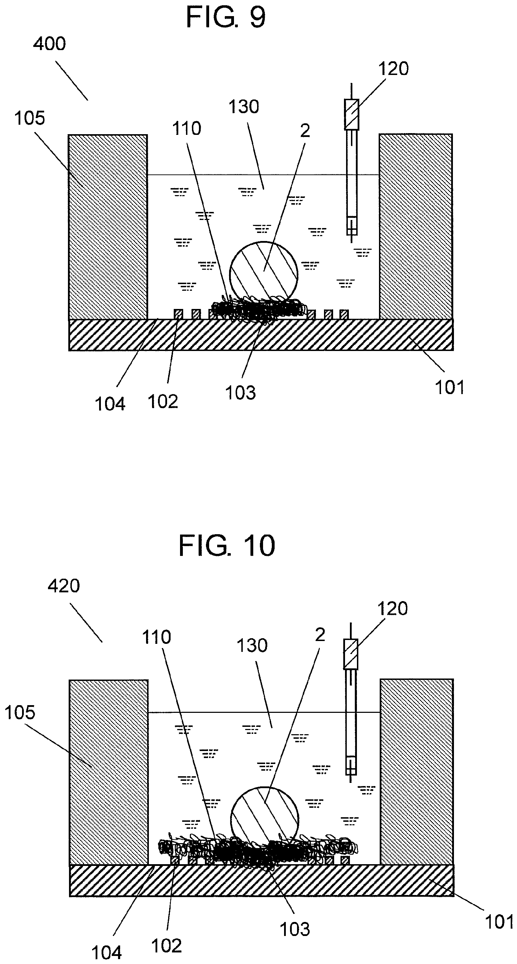

FIG. 9 is a vertical cross-sectional view of examination device 400 for substance 2 of biological origin according to the present exemplary embodiment. The present exemplary embodiment differs from the first exemplary embodiment in that separator 110 made of a porous material or a fibrous material is disposed above placing area 103 for placing substance 2 of biological origin.

Examples of a porous material to be used include hydrogel, silica gel, and the like.

Examples of a fibrous material to be used include glass fiber, inorganic nanofiber, organic nanofiber, and nitrocellulose.

According to the above configuration, separator 110 prevents direct contact of substance 2 of biological origin with the surface of base 101. On the other hand, culture solution 130 for maintaining an appropriate state of substance 2 of biological origin is a fluid, and thus culture solution 130 passes through separator 110, and is smoothly supplied to the vicinity of substance 2 of biological origin. Similarly, wastes generated by substance 2 of biological origin through metabolism can be quickly removed from the vicinity of substance 2 of biological origin.

FIG. 10 is a vertical cross-sectional view of examination device 420 for substance 2 of biological origin according to the present exemplary embodiment. Separator 110 made of a porous material or a fibrous material is formed not only above placing area 103 at which substance 2 of biological origin is placed, but also above monitor electrodes 102.

Accordingly, the upper portions of monitor electrodes 102 are covered with the porous material or the fibrous material. Here, separator 110 has spaces inside, and thus allows culture solution 130 to pass through, but does not prevent diffusion of dissolved oxygen around a substance of biological origin.

The above configuration prevents substance 2 of biological origin from being in direct contact with monitor electrodes 102. Accordingly, the amount of dissolved oxygen can be measured more accurately, and also inhibits wastes from substance 2 of biological origin from adhering to monitor electrode 102.

### FIFTH EXEMPLARY EMBODIMENT

The following describes examination device 500 for substance 2 of biological origin according to a fifth exemplary embodiment, with reference to the drawings. In the present exemplary embodiment, the same numerals are assigned to an equivalent configuration to that in the first exemplary embodiment, and a detailed description thereof is omitted.

FIG. 11 is a vertical cross-sectional view of examination device 500 for substance 2 of biological origin according to the present exemplary embodiment. The present exemplary embodiment differs from the first exemplary embodiment in that monitor electrodes 102 are formed on the surface of base 101, and separator 110 made of a porous material or a fibrous material is disposed above monitor electrodes 102. Separator 110 is not in contact with placing area 103, and is in contact with monitor electrodes 102. Separator 110 is the same as or similar to that of the fourth exemplary embodiment, and thus a detailed description thereof is omitted.

Separator 110 has spaces inside, and thus allows culture solution 130 to pass through, but does not prevent diffusion of dissolved oxygen around substance 2 of biological origin.

The above configuration allows measurement of a concentration of dissolved oxygen while inhibiting substance 2 of biological origin from being in direct contact with monitor electrodes 102. Separator 110 according to the present exemplary embodiment is not used as means for placing substance 2 of biological origin, but used to inhibit substance 2 of biological origin from being in contact with monitor electrodes 102.

As described above, in the present exemplary embodiment, monitor electrodes 102, 202, and 302 are disposed separate from substance 2 of biological origin by different distances. Accordingly, physicochemical changes can be measured at the positions of monitor electrodes 102, 202, and 302, without the necessity of moving monitor electrodes to make measurements, as with a conventional technique. As a result, physicochemical changes around substance 2 of biological origin can be measured with ease in a spatially resolved manner.

### INDUSTRIAL APPLICABILITY

The examination device for a substance of biological origin according to the exemplary embodiments is useful to examine and analyze the activity states of substances of biological origin typified by a cell, tissue, an embryo, and the like.

### REFERENCE MARKS IN THE DRAWINGS

2 substance of biological origin
100, 200, 210, 220, 300, 320, 330, 340, 360, 400, 420, 500 examination device
101 base
102, 102a, 102b, 102c, 202, 202a, 202b, 202c, 202d, 302 monitor electrode
103 placing area
104 placing surface
105 wall portion
106 well
107 opposite surface
108 first through hole
109 second through hole
110 separator
120 reference electrode
130 culture solution
302a, 302b, 302c distance

## Claims

1. A combination of at least one instrumentation amplifier and an examination device for a substance of biological origin, the examination device comprising:
a base (101);
a placing area (103) for placing the substance of biological origin, the placing area being on a first surface of the base;
a reference electrode (120); and
a first monitor electrode (102a) and a second monitor electrode (102b) which are disposed on the first surface of the base (101), the first monitor electrode (102a) and the second monitor electrode (102b) arranged as individually connected to the at least one instrumentation amplifier and arranged to individually measure physicochemical change occurring around the substance (2) of biological origin as current or potential difference between the reference electrode (120) and each of the monitoring electrodes (102a, 102b), **characterised in that** a shortest distance from a center of the placing area to the first monitor electrode (102a) and a shortest distance from the center of the placing area to the second monitor electrode (102b) are different, and
wherein the first monitor electrode (102a) and the second monitor electrode (102b) are further arranged to individually measure the physicochemical change at different distances from the substance (2) of biological origin placed at the placing area (103).

2. The combination of at least one instrumentation amplifier and an examination device according to claim 1,
wherein the base (101) has, in the placing area (103), a first through hole penetrating (108) through the base (101) from the first surface to a second surface on an opposite side of the base (101) from the first surface.

3. The combination of at least one instrumentation amplifier and an examination device according to claim 2,
wherein the base (101) has, in an area other than the placing area (103), a plurality of second through holes (109) penetrating through the base (101) from the first surface to the second surface.

4. The combination of at least one instrumentation amplifier and an examination device according to claim 1,
wherein the base (101) has, in an area other than the placing area (103), a plurality of second through holes (109) penetrating through the base (101) from the first surface to a second surface on an opposite side of the base from the first surface.

5. The combination of at least one instrumentation amplifier and an examination device according to claim 1, the examination device further comprising
a separator (110) which is disposed above the placing area (103), and includes a porous material or a fibrous material.

6. The combination of at least one instrumentation amplifier and an examination device according to claim 5,
wherein the separator (110) is further disposed above the first monitor electrode (102a) and the second monitor electrode (102b).

7. The combination of at least one instrumentation amplifier and an examination device according to claim 1, the examination device further comprising
a separator (110) which is disposed above the first monitor electrode (102a) and the second monitor electrode (102b), and includes a porous material or a fibrous material.

8. The combination of at least one instrumentation amplifier and an examination device according to claim 1, the examination device further comprising
a wall portion (105) surrounding a perimeter of the base (101).

9. The combination of at least one instrumentation amplifier and an examination device according to claim 1,
wherein the first monitor electrode (102a) and the second monitor electrode (102b) are circular when viewed in a direction facing the first surface.

## Patentansprüche

1. Kombination aus zumindest einem Instrumentenverstärker und einer Vorrichtung zur Untersuchung einer Substanz biologischen Ursprungs, wobei die Untersuchungsvorrichtung Folgendes umfasst:
eine Basis (101);
einen Ablagebereich (103) zum Ablegen der Substanz biologischen Ursprungs, wobei sich der Ablagebereich auf einer ersten Fläche der Basis befindet; eine Bezugselektrode (120); und
eine erste Überwachungselektrode (102a) und eine zweite Überwachungselektrode (102b), die auf der ersten Fläche der Basis (101) vorhanden sind, wobei die erste Überwachungselektrode (102a) und die zweite Überwachungselektrode (102b) so angeordnet sind, dass sie einzeln mit dem zumindest einen Instrumentenverstärker verbunden und so angeordnet sind, dass sie einzeln die physikalisch-chemische Veränderung messen, die um die Substanz (2) biologischen Ursprungs herum als Strom- oder Potentialdifferenz zwischen der Referenzelektrode (120) und jeder der Überwachungselektroden (102a, 102b) auftritt,
**dadurch gekennzeichnet, dass**
ein kürzester Abstand von einer Mitte des Ablagebereichs zur ersten Überwachungselektrode (102a) und ein kürzester Abstand von der Mitte des Ablagebereichs zur zweiten Überwachungselektrode (102b) unterschiedlich sind, und
wobei die erste Überwachungselektrode (102a) und die zweite Überwachungselektrode (102b) ferner so angeordnet sind, dass sie die physikalisch-chemische Veränderung in unterschiedlichen Abständen von der Substanz (2) biologischen Ursprungs einzeln messen, die auf der Ablagefläche (103) platziert ist.

2. Kombination aus zumindest einem Instrumentenverstärker und einer Vorrichtung zur Untersuchung nach Anspruch 1,
wobei die Basis (101) in dem Ablagebereich (103) ein erstes Durchgangsloch (108) aufweist, das von der ersten Fläche zu einer zweiten Fläche auf einer gegenüberliegenden Seite der Basis (101) von der ersten Fläche durch die Basis (101) hindurchgeht

3. Kombination aus zumindest einem Instrumentenverstärker und einer Vorrichtung zur Untersuchung nach Anspruch 2,
wobei die Basis (101) in einem anderen Bereich als dem Ablagebereich (103) eine Mehrzahl von zweiten Durchgangslöchern (109) aufweist, die von der ersten Oberfläche zur zweiten Oberfläche durch die Basis (101) hindurchgehen.

4. Kombination aus zumindest einem Instrumentenverstärker und einer Vorrichtung zur Untersuchung nach Anspruch 1,
wobei die Basis (101) in einem anderen Bereich als dem Ablagebereich (103) eine Mehrzahl von zweiten Durchgangslöchern (109) aufweist, die von der ersten Fläche zu einer zweiten Fläche auf einer gegenüberliegenden Seite der Basis (101) von der ersten Fläche durch die Basis hindurchgehen.

5. Kombination aus zumindest einem Instrumentenverstärker und einer Vorrichtung zur Untersuchung nach Anspruch 1, wobei die Untersuchungsvorrichtung ferner umfasst
einen Abscheider (110), der über dem Ablagebereich (103) angeordnet ist und ein poröses oder faseriges Material enthält.

6. Kombination aus zumindest einem Instrumentenverstärker und einer Vorrichtung zur Untersuchung nach Anspruch 5,
wobei der Abscheider (110) ferner oberhalb der ersten Überwachungselektrode (102a) und der zweiten Überwachungselektrode (102b) angeordnet ist.

7. Kombination aus zumindest einem Instrumentenverstärker und einer Vorrichtung zur Untersuchung nach Anspruch 1, wobei die Untersuchungsvorrichtung ferner umfasst
einen Abscheider (110), der über der ersten Überwachungselektrode (102a) und der zweiten Überwachungselektrode (102b) angeordnet ist und ein poröses oder faseriges Material enthält.

8. Kombination aus zumindest einem Instrumentenverstärker und einer Vorrichtung zur Untersuchung nach Anspruch 1, wobei die Untersuchungsvorrichtung ferner umfasst
einen Wandabschnitt (105), der einen Umfang der Basis (101) umgibt.

9. Kombination aus zumindest einem Instrumentenverstärker und einer Vorrichtung zur Untersuchung nach Anspruch 1,
wobei die erste Überwachungselektrode (102a) und die zweite Überwachungselektrode (102b) kreisförmig sind, wenn sie in einer der ersten Oberfläche zugewandten Richtung betrachtet werden.

## Revendications

1. Combinaison d'au moins un amplificateur d'instrumentation et d'un dispositif d'examen pour une substance d'origine biologique, le dispositif d'examen comprenant :
une base (101) ;
une zone de placement (103) pour placer la substance d'origine biologique, la zone de placement étant sur une première surface de la base ;
une électrode de référence (120) ; et
une première électrode de surveillance (102a) et une deuxième électrode de surveillance (102b) qui sont disposées sur la première surface de la base (101), la première électrode de surveillance (102a) et la deuxième électrode de surveillance (102b) étant agencées de façon à être connectées individuellement à l'au moins un amplificateur d'instrumentation et agencées de façon à mesurer individuellement un changement physico-chimique se produisant autour de la substance (2) d'origine biologique sous la forme d'une différence de courant ou de potentiel entre l'électrode de référence (120) et chacune des électrodes de surveillance (102a, 102b),
**caractérisée en ce que** la distance la plus courte entre le centre de la zone de placement et la première électrode de surveillance (102a), et la distance la plus courte entre le centre de la zone de placement et la deuxième électrode de surveillance (102b), sont différentes, et
dans laquelle la première électrode de surveillance (102a) et la deuxième électrode de surveillance (102b) sont en outre agencées de façon à mesurer individuellement le changement physico-chimique à des distances différentes de la substance (2) d'origine biologique placée au niveau de la zone de placement (103).

2. Combinaison d'au moins un amplificateur d'instrumentation et d'un dispositif d'examen selon la revendication 1,
dans laquelle la base (101) a, dans la zone de placement (103), un premier trou traversant (108) pénétrant à travers la base (101) depuis la première surface jusqu'à une deuxième surface sur un côté opposé de la base (101) par rapport à la première surface.

3. Combinaison d'au moins un amplificateur d'instrumentation et d'un dispositif d'examen selon la revendication 2,
dans laquelle la base (101) a, dans une zone autre que la zone de placement (103), une pluralité de deuxièmes trous traversants (109) pénétrant à travers la base (101) depuis la première surface jusqu'à la deuxième surface.

4. Combinaison d'au moins un amplificateur d'instrumentation et d'un dispositif d'examen selon la revendication 1,
dans laquelle la base (101) a, dans une zone autre que la zone de placement (103), une pluralité de deuxièmes trous traversants (109) pénétrant à travers la base (101) depuis la première surface jusqu'à une deuxième surface sur un côté opposé de la base par rapport à la première surface.

5. Combinaison d'au moins un amplificateur d'instrumentation et d'un dispositif d'examen selon la revendication 1, dans laquelle le dispositif d'examen comprend en outre
un séparateur (110) qui est disposé au-dessus de la zone de placement (103), et qui comprend un matériau poreux ou un matériau fibreux.

6. Combinaison d'au moins un amplificateur d'instrumentation et d'un dispositif d'examen selon la revendication 5,
dans laquelle le séparateur (110) est en outre disposé au-dessus de la première électrode de surveillance (102a) et de la deuxième électrode de surveillance (102b).

7. Combinaison d'au moins un amplificateur d'instrumentation et d'un dispositif d'examen selon la revendication 1, dans laquelle le dispositif d'examen comprend en outre
un séparateur (110) qui est disposé au-dessus de la première électrode de surveillance (102a) et de la deuxième électrode de surveillance (102b), et qui comprend un matériau poreux ou un matériau fibreux.

8. Combinaison d'au moins un amplificateur d'instrumentation et d'un dispositif d'examen selon la revendication 1, dans lequel le dispositif d'examen comprend en outre
une partie de paroi (105) entourant le périmètre de la base (101).

9. Combinaison d'au moins un amplificateur d'instrumentation et d'un dispositif d'examen selon la revendication 1,
dans laquelle la première électrode de surveillance (102a) et la deuxième électrode de surveillance (102b) sont circulaires quand elles sont regardées dans une direction faisant face à la première surface.
